# EUROPEAN PATENT SPECIFICATION

(11) **EP 0 732 949 B1**
(45) Date of publication and mention of the grant of the patent: **26.03.2003**
(21) Application number: 94907094.0
(22) Date of filing: 17.12.1993
(51) Int. Cl.: A61M 1/36

(54) **SYSTEM FOR CARDIAC PROCEDURES**
SYSTEM FÜR HERZOPERATIONEN
SYSTEME POUR INTERVENTIONS CARDIAQUES

(43) Date of publication of application: 25.09.1996
(73) Proprietor: HEARTPORT, INC., Redwood City, California 94063 (US)
(72) Inventor: Stevens, John H., Palo Alto, CA 94303 (US)
(74) Representative: Harrison, David Christopher
(86) International application number: US9312323
(87) International publication number: WO95016476

(56) References cited:
- EP-A- 0 350 302
- EP-A- 0 357 338
- WO-A-91/01689
- WO-A-92/17118
- WO-A-93/01768
- WO-A-95/15192
- US-A- 4 689 041
- DATABASE WPI Week 8134, Derwent Publications Ltd., London, GB; AN 81-H8732D & SU,A,782 808 (DOCTORS ADVANC INST) 1 December 1980

## Description

This invention relates to systems for performing cardiac procedures and particularly endovascular cardiac procedures such as the placement of or the removal and replacement of heart valves.

There are two major categories of heart valve disease, namely, stenosis, which is an obstruction to forward blood flow caused by a heart valve, and regurgitation, which is the retrograde leakage of blood through a heart valve.

When it is necessary to repair or replace a malfunctioning heart valve within a patient, heretofore the repair or replacement has been accomplished by a major open-heart surgical procedure, requiring general anesthesia and full cardiopulmonary by-pass with complete cessation of cardiopulmonary activity. Such surgery usually includes about three weeks of hospitalization and months of recuperation time for the patient. The average mortality rate with this type of procedure is about five to six percent, and the complication rate is substantially higher. Descriptions of open-heart procedures for replacing heart valves can be found in Gibbon's Surgery of the Chest, 5th Ed., David C. Sabiston, Jr., M.D., Frank D. Spencer, M.D., 1990, Vol. II, Ch. 52, pp. 1566-1596, and Textbook of Interventional Cardiology, Eric J. Topol, 1990, Chs. 43-44, pp. 831-867.

Endovascular surgical procedures on the heart have been developed recently which, in contrast to open-heart surgical procedures, may have a reduced mortality rate, may require only local anesthesia, and may necessitate only a few days of hospitalization. However, the range of applications of endovascular heart procedures other than those of the coronary arteries, such as angioplasty and atherectomy, has been limited.

Some progress has been made in developing endovascular procedures involving the heart valves. For example, for patients with severe stenotic valve disease, who are too compromised to tolerate open- heart surgery to replace the heart valve as described above, surgeons have attempted endovascular balloon aortic or mitral valvuloplasty. These procedures involve endovascularly advancing a balloon dilatation catheter into the patient's vasculature until the balloon of the catheter is positioned'between the valve leaflets and then inflating the balloon to split the commissures in a diseased valve with commissural fusion and to crack calcific plaques in a calcified stenotic valve. However, this method may provide only partial and temporary relief for a patient with a stenotic valve. The rapid restenosis following balloon aortic valvuloplasty has led to virtual abandonment of this procedure as a treatment of the diseased aortic valve.

An endovascular treatment regimen for regurgitant heart valves, which involves valve supplantation, has been disclosed in the patent literature, but apparently the procedure has not been clinically practised. In this procedure, it is conceived that an elongated catheter is used to insert a mechanical valve into the lumen of the aorta via entry through a distal artery, for example, the brachial or femoral artery. One such mechanical valve is described in US-A-4,056,854 (Boretos *et al*.) that is designed to be positioned against the artery wall during forward flow, as compared to the mid-center position of the valve described in US-A-3,671,979 (Moulopoulos). The valve positioned against the arterial wall is intended to reduce the stagnation of blood flow and consequent thrombus and emboli formation compared to a valve at mid-center position. The mechanical valves previously described require an elongated mounting catheter extending out of the arterial entry point to maintain the position of the valve in the descending aorta. These valves would be expected to present several problems. The valves do not provide a permanent or internalized system. Furthermore, since both involve a mechanical valve, which predisposes the patient to thrombus formation and emboli, long term anticoagulant therapy is required. A serious complication of long term anticoagulant therapy is intracranial hemorrhage. Finally, the supplemental valve is placed downstream from both the normal valve position and the coronary ostia, so normal heart and coronary artery hemodynamics are not restored.

The descriptive terms upstream and downstream, when used herein in relation to the patient's vasculature, refer to directions closer to and further from the heart, as applicable. The terms proximal and distal, when used herein in relation to instruments used in the procedure, refer to directions closer to and farther away from the operator performing the procedure.

What have been needed and heretofore unavailable are methods and systems for satisfactorily performing various endovascular procedures, particularly those suitable for heart valve placement or removal and replacement, which do not require a thoracotomy. The present invention satisfies these and other needs.

The present invention is directed to a system for an endovascular approach for preparing a patient's heart for cardiac procedures which does not require a grossly invasive thoracotomy. The system of the present invention is defined in claim 1.

The endovascular system of the invention includes an elongated catheter having proximal and distal ends and an occluding member on a distal portion of the catheter adapted to occlude a patient's ascending aorta. The catheter has an inner lumen extending within the catheter to a port in the distal end of the catheter. The catheter is adapted to be inserted into the patient's arterial system (*e.g.* through the femoral or brachial arteries) and to be advanced to the ascending aorta where the occluding member is expanded to occlude the aorta at that location. In so doing the left ventricle of the heart and an upstream portion of the ascending aorta are separated from the rest of the patient's arterial system. This catheter thus constitutes an endovascularly inserted, internal vascular clamp, similar in function to the external "cross-clamp" used in open-surgical procedures. The internal clamp is less traumatic to the clamped vessel, and provides a lumen or working channel through which instruments or fluids may be passed into or withdrawn from the area upstream of the end of the distal end of the clamp. The occluding member on the elongated catheter should be dimensioned so that upon expansion it will be located downstream from the ostia for the coronary arteries and upstream from the brachiocephalic artery so as to avoid blocking these arteries. The inner lumen of the occluding catheter is dimensioned to allow for the passage therethrough of instruments for the performing the cardiac procedure.

Also included with the system is a cardiopulmonary by-pass system which withdraws blood from the patient's venous system, *e.g.* the femoral or jugular vein, removes CO₂ from and adds oxygen to the withdrawn blood, and then returns the oxygenated blood to the patient's arterial system, *e.g.* the femoral or brachial artery. The system is also provided with means to deliver a fluid containing cardioplegic material (*e.g.* an aqueous solution of KCI and/or magnesium procaine and the like) through the coronary arteries so as to paralyze the myocardium.

It is also preferred to depressurize the left atrium by venting the left atrium via a catheter placed in the pulmonary artery. This catheter may actually occlude the pulmonary artery to further prevent blood from flowing to the lungs. With the heart paralyzed, the expandable member of the aortic catheter expanded within the ascending aorta, and the cardiopulmonary by-pass operating, the heart is prepared for a cardiac procedure. While a particularly attractive feature of the invention is that it prepares the heart for an endovascular procedure, the invention can also be used to prepare the heart for conventional open-heart surgery via a thoracotomy. It should also be noted that, if during an endovascular cardiac procedure in accordance with the invention it becomes necessary to perform an open-heart procedure, the patient is already fully prepared for the open-heart procedure. All that is necessary is to perform a thoracotomy to expose the patient's heart for the conventional surgical procedure. So much is known from our earlier WO-A-93/017 68.

In the present invention directed to endovascular cardiac procedures, the occlusion catheter is adapted to deliver a new valve, and allow the securing of the new valve at the desired location. In these procedures, the expanded expandable member on the distal end of the occlusion catheter firmly secures the distal end of the catheter within the aorta to allow for the accurate guidance of instruments to be used during the procedure.

By partitioning the arterial system with the elongated aortic catheter in this manner, a body of clear fluid can be maintained in the aortic region upstream from the expanded distal end of the aortic catheter to facilitate the imaging, *e.g.* angioscopic observation, of the cardiac procedure. A continual flow of clear fluid may be directed to the surgical field in order to maintain fluid clarity sufficient for imaging the site during the procedure. The pressure of the body of irrigation fluid at the surgical site can be maintained at a level equal to or higher than the fluid pressure in the patient's left atrium to prevent the intrusion of blood from the left atrium into the left ventricle, which can interfere with the imaging. The temperature of the irrigating fluid should be about 4° C in order to reduce myocardial oxygen demand.

The system further includes grasping means for holding the aortic valve as it is being severed by the cutting means.

In order to deliver cardioplegic fluids to the myocardium, it is presently preferred to carry out retrograde perfusion of the coronary circulation. Using this technique, a physician will percutaneously introduce a catheter through a major vein, *e.g.* the right internal jugular vein, and advance the catheter in the venous system until the distal end of the catheter extends into the coronary sinus through the discharge opening thereof in the right atrium. Preferably, the catheter has an inflatable balloon on the distal end thereof, such as shown in US-A-4,689,041, 4,943,277, and 5,021,045. When inflated, the balloon blocks the discharge opening of the coronary sinus to preclude loss of cardioplegic fluid therefrom. With the discharge opening of the coronary sinus blocked off, aqueous liquid or other fluid containing cardioplegic material is delivered through the catheter into the coronary sinus at sufficient pressure so that it passes into the myocardium via the capillary bed between the venous and arterial systems therein so as to paralyze the entire myocardium. Typically, cardioplegic solution pressure within the coronary sinus should be less than 13.32 kPa (100 mm Hg) to avoid tissue damage. After passing through the myocardium, the cardioplegic liquid will pass through the coronary arteries in a retrograde fashion to be discharged through the coronary ostia into the upstream portion of the ascending aorta. The cardioplegic fluid which discharges from the coronary ostia will initially be very opaque due to blood being flushed out of the coronary circulation, but eventually the fluid will become clear and may be conveniently used to form and maintain the body of clear fluid at the surgical site to facilitate the imaging thereof during the procedure. In some instances, cardioplegic liquid may instead be delivered through the coronary arteries in an antegrade fashion, either via catheters placed through the coronary ostia into the coronary arteries or by delivery via the aortic catheter directly into the aortic root.

The left atrium is preferably decompressed by one of two methods. The first involves a catheter passing into the pulmonary trunk. The catheter described is advanced through the patient's venous system, *e.g.* through the right internal jugular vein, through the right atrium and the right ventricle, and into the pulmonary trunk. This catheter can vent fluid from the pulmonary trunk via an inner lumen extending from its distal port to a port in its proximal end located outside the patient. It may be advantageous to nave an inflatable member located at the distal end of the ventilation catheter. The inflatable member is dimensioned so that upon inflation it will block the pulmonary trunk while simultaneously venting the trunk through the inner lumen of the catheter, which extends through the catheter from a port in its distal end to a port in its proximal end located outside of the patient.

In an alternative method, as described in US-A-4,889,137 (Kolobow) a catheter is advanced in essentially the same manner as that described above until the distal end is within the pulmonary trunk. As described in this patent, springs or other means are provided on the exterior of the catheter at the locations where the catheter will extend through the pulmonary and tricuspid valves in order to hold the valves at least partially open and thereby vent the pulmonary artery and decompress the left atrium.

The occluding aortic catheter with an expandable occluding member on the distal end, coupled with cardiopulmonary by-pass, cardioplegia, and decompression of the left atrium, provides for a unique intravascular approach to the supply and securing of a prosthetic valve in an approach which does not require invasive thoracic or abdominal surgery. Moreover, as mentioned, the system may even be employed in conventional open-heart procedures. In addition, the system may be used in minimally invasive cardiac procedures under thoracoscopic guidance, working through incisions in the patient's chest from outside the patient's body. In these instances the occluding catheter need not have an inner lumen for delivery of fluids and the like. These and other advantages of the invention will become more apparent from the following detailed description of the invention when taken in conjunction with the accompanying exemplary drawings, in which:
Fig. 1 schematically illustrates a cardiac access system embodying features of the invention.
Figure 2 is an enlarged view, partially in section, of the occluding catheter shown in Fig. 1 disposed within the ascending aorta.
Fig. 3 is a transverse cross-sectional view of the occluding catheter shown in Fig. 2 taken along the lines 3-3.
Fig. 4 is an enlarged view, partially in section, of the cardioplegia delivery catheter and the pulmonary venting catheter shown in Fig. 1.
Fig. 5 is an elevational view, partially in section, of the occluding catheter shown in Fig. 2 schematically illustrating the removal of an aortic heart valve.
Fig. 6 schematically illustrates the introduction of a prosthetic valve into the region of the ascending aorta from which the original heart valve had been removed.
Fig. 7 schematically illustrates securing a mounting skirt on the prosthetic valve to the wall of the ascending aorta.
Fig. 8 schematically illustrates securing the upper extensions of the valve to the aortic wall.
Fig. 9 schematically illustrates an alternate means for removing a heart valve.
Fig. 10 is an enlarged perspective view of the cutting member of the catheter shown in Fig. 9.
Fig. 11 schematically illustrates another alternate means for removing a heart valve.
Figs. 12 and 13 schematically illustrate an alternate embodiment of a valve introducing device and the method of discharging a prosthetic or replacement valve.
Fig. 14 schematically represents in an elevational view a prosthetic heart valve.
Fig. 15 is a top view of the prosthetic heart valve shown in Fig. 14.

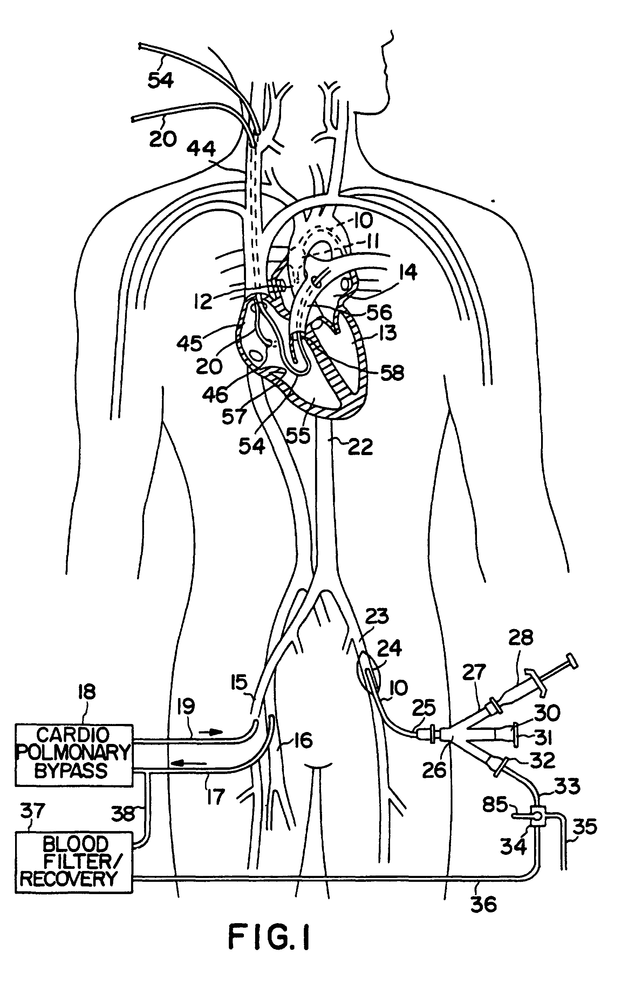

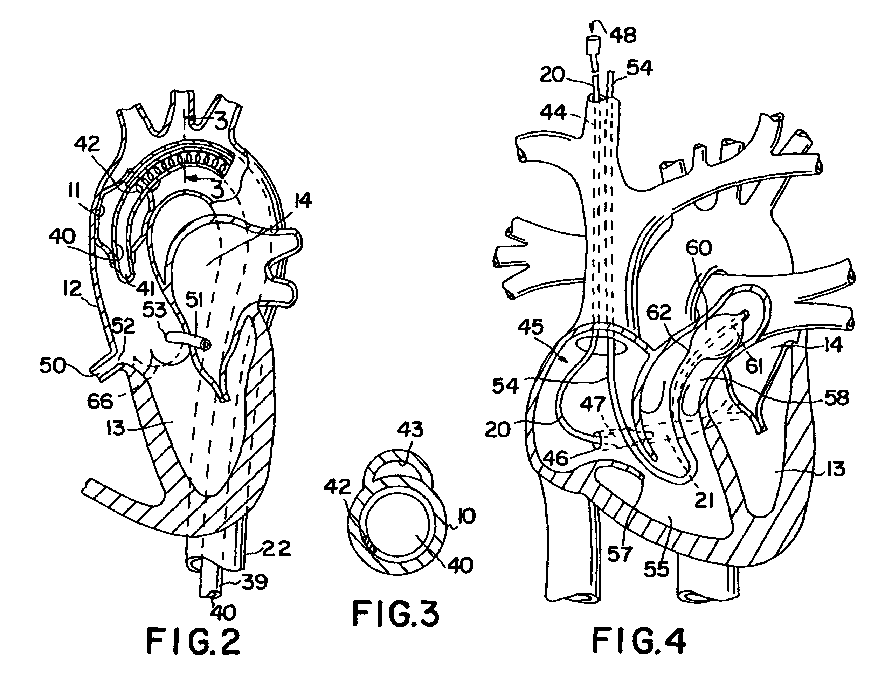

Reference is made to Fig. 1 which schematically illustrates the overall cardiac accessing system of the invention and the individual components thereof. The accessing system includes an elongated aortic occlusion or delivery catheter 10 which has an expandable member 11 on a distal portion of the catheter which, when inflated as shown, occludes the ascending aorta 12 to separate the left ventricle 13 and upstream portion of the ascending aorta from the rest of the patient's arterial system and securely positions the distal end of the catheter within the ascending aorta. A cardiopulmonary by-pass system 18 removes venous blood from the femoral vein 16 through the blood withdrawal catheter 17 as shown, removes CO₂ from the blood, oxygenates the blood, and then returns the oxygenated blood to the patient's femoral artery 15 through the return catheter 19 at sufficient pressure so as to flow throughout the patient's arterial system except for the portion blocked by the expanded occluding member 11 on the aortic occluding catheter 10. A retrograde cardioplegia balloon catheter 20 is disposed within the patient's venous system with the distal end of the catheter extending into the coronary sinus 21 (shown in Fig. 4) to deliver a fluid containing cardioplegic agents to the myocardium in a retrograde manner through the patient's coronary venous system to paralyze the entire myocardium.

The elongated occluding catheter 10 extends through the descending aorta to the left femoral artery 23 and out of the patient through a cut down 24. The proximal extremity 25 of the catheter 10 which extends out of the patient is provided with a multi-arm adapter 26 with one arm 27 adapted to receive an inflation device 28. The adapter 26 is also provided with a second arm 30 with main access port 31 through which passes instruments, a valve prosthesis, an angioscope, irrigation fluid and the like. A third arm 32 connected to by-pass line 33 is provided to direct blood, irrigation fluid, and the like to or from the system. A suitable valve 34 is provided to open and close the by-pass line 33 and direct the fluid passing through the by-pass line to a discharge line 35 or a line 36 to a blood filter and recovery unit 37. A return line 38 may be provided to return any filtered blood, which will be described hereinafter, to the cardiopulmonary by-pass system 18 or other blood conservation system.

The details of the aortic occlusion catheter 10 and the disposition of the distal extremity thereof within the aorta are best illustrated in Figs. 2 and 3. As indicated, the catheter 10 includes an elongated catheter shaft 39 which has a first inner lumen 40 in fluid communication with the main access port 31 in the second arm of the adapter 26 and is adapted to facilitate the passage of instruments, a valve prosthesis, an angioscope, irrigation fluid, and the like therethrough and out the distal port 41 in the distal end thereof. A supporting coil 42 may be provided in the distal portion of the first inner lumen 40 to prevent the catheter shaft 39 from kinking as it is advanced through the aortic arch. The shaft 39 is also provided with a second inner lumen 43 which is in fluid communication with the interior of the occluding balloon 11.

A retrograde cardioplegia balloon catheter 20, which is shown in more detail in Fig. 4, is introduced into the patient's venous system through the right internal jugular vein 44 and is advanced through the right atrium 45 and into the coronary sinus 21 through the coronary sinus discharge opening 46 in the right atrium. The retrograde catheter 20 is provided with a balloon 47 on a distal portion of the catheter 20 which is adapted to occlude the coronary sinus 21 when inflated. A liquid containing a cardioplegic agent, e.g. an aqueous KCI solution, is introduced into the proximal end 48 of the catheter 20, which extends outside of the patient, under sufficient pressure so that the fluid containing the cardioplegic agent can be forced to pass through the coronary sinus 21, through the capillary beds (not shown) in the patient's myocardium, through the coronary arteries 50 and 51 and ostia 52 and 53 associated with the respective coronary arteries into the blocked off portion of the ascending aorta 12 as shown.

A pulmonary venting catheter 54 is also shown in Fig. 4 disposed within the right internal jugular vein 44 and extending through the right atrium 45 and right ventricle 55 into the pulmonary trunk 56. The catheter 54 passes through tricuspid valve 57 and pulmonary valve 58. An inflatable occluding balloon 60 may be provided as shown on a distal portion of the pulmonary venting catheter 54 which is inflated to occlude the pulmonary trunk 56 as shown. The pulmonary venting catheter 54 has a first inner lumen 61 which extends from the distal end of the catheter to the proximal end of the catheter which vents fluid from the pulmonary trunk 56 to outside the patient's body either for discharge or for passage to the blood recovery unit and thereby decompresses the left atrium 14 through the pulmonary capillary beds (not shown). The catheter 54 has a second inner lumen 62 which is adapted to direct inflation fluid to the interior of the inflatable balloon 60.

To set up the cardiac access system, the patient is initially placed under light general anesthesia. The withdrawal catheter 17 and the return catheter 19 of the cardiopulmonary by-pass system 18 are percutaneously introduced into the right femoral vein 16 and the right femoral artery 15, respectively. An incision 24 is also made in the left groin to expose the left femoral artery 23 and the aortic occluding catheter 10 is inserted into the left femoral artery through an incision therein and advanced upstream until the balloon 11 on the distal end of the occluding catheter 10 is properly positioned in the ascending aorta 12. Note that by-pass could similarly be established in the left groin and the aortic occlusion catheter put into the right femoral artery. The retrograde perfusion catheter 20 is percutaneously inserted by a suitable means such as the Seldinger technique into the right interior jugular vein 44 and advanced into the right atrium 45 and guided through the discharge opening 46 into the coronary sinus 21.

The pulmonary venting catheter 54 is advanced through the right internal jugular vein 44, the right atrium 45, and right ventricle 55, and into the pulmonary trunk 56. The occluding balloon 60 may be inflated if necessary by inflation with fluid passing through the lumen 62 to block the pulmonary trunk 56 and vent blood therein through the lumen 61 where it is discharged through the proximal end of the catheter which extends outside of the patient. The venting of the pulmonary trunk 56 results in the decompressing of the left atrium 14. In the alternative, the venting catheter 54 may be provided with means on the exterior thereof, such as expanded coils as described in U.S. Patent 4,889,137 (Kolobow), which hold open the tricuspid and pulmonary valves and perform the same function of decompressing the left atrium. See also the article written by F. Rossi *et al.* in the Journal of Thoracic Cardiovascular Surgery, 1990; 100:914-921, entitled "Long-Term Cardiopulmonary Bypass By Peripheral Cannulation In A Model Of Total Heart Failure",

The operation of the cardiopulmonary by-pass unit 18 is initiated to withdraw blood from the femoral vein 16 through catheter 17, remove CO₂ from and add oxygen to the withdrawn blood and then pump the oxygenated blood through the return catheter 19 to the right femoral artery 15. The balloon 11 may then be inflated to occlude the ascending aorta 12, causing the blood pumped out of the left ventricle (until the heart stops beating due to the cardioplegic fluid as discussed hereinafter) to flow through the discharge port 41 into the first inner lumen 40 of the occluding catheter. The blood flows through the inner lumen 40 and out the third arm 32 of the adapter 26 into the by-pass line 33 and then into the blood filter and blood recovery unit 37 through the valve 34 and line 36. For blood and irrigation fluids containing debris and the like, the position of the valve 34 may be changed to direct the fluid through the discharge line 35.

The balloon 47 on the distal extremity of the retroperfusion catheter 20 is inflated to occlude the coronary sinus 21 to prevent fluid loss through the discharge opening 46 into the right atrium 45. A liquid containing a cardioplegic agent such as KCI is directed through the catheter 20 into the coronary sinus 21 and the pressure of the cardioplegic fluid within the coronary sinus 21 is maintained sufficiently high (*e.g.* 5.33 kPa (40 mm Hg)) so that the cardioplegic fluid will pass though the coronary veins, crossing the capillary beds to the coronary arteries 50 and 51 and out the ostia 52 and 53. However, cardioplegic fluid level is not increased far above 13.32 kPa (100 mm Hg). Once the cardioplegic fluid passes through the capillary beds in the myocardium, the heart very quickly stops beating. At that point the myocardium is paralyzed and has very little demand for oxygen and can be maintained in this state for long periods of time with minimal damage.

With the cardiopulmonary by-pass system in operation, the heart completely paralyzed and not pumping, the left atrium decompressed and the ascending aorta blocked by the inflated balloon 11 on the occluding catheter 10, the heart is appropriately prepared for a cardiac procedure.

Inflation of the inflatable member 11 on the distal end of the delivery catheter 10 fixes the distal end of the occluding catheter 10 within the ascending aorta 12 and isolates the left ventricle 13 and the upstream portion of the ascending aorta from the rest of the arterial system downstream from the inflatable member. The passage of any debris or emboli, solid or gaseous, generated during a cardiovascular procedure to regions downstream from the site would be precluded by the inflated balloon 11. Fluid containing debris or emboli can be removed from the region between the aortic valve and the occluding balloon 11 through the inner lumen 40 of catheter 10. A clear, compatible fluid, *e.g.* an aqueous based fluid such as saline delivered through the inner lumen 40 or the cardioplegic fluid discharging from the coronary ostia 52 and 53, may be maintained in the region wherein the cardiovascular procedure is to be performed to facilitate use of an angioscope or other imaging means that allows for direct observation of the cardiac procedure. Preferably, the fluid pressure in the left ventricle 13 is maintained sufficiently higher than that in the left atrium to prevent blood from the left atrium from seeping into the left ventricle and interfering with the observation of the procedure. The inner lumen 40 is dimensioned to allow for the passage of instruments used during the cardiac procedure such as a tissue cutter, an angioscope, and tubes used for infusing irrigation fluid and for aspirating debris, thrombus and the like, and for the introduction of a prosthetic device, such as a heart valve.

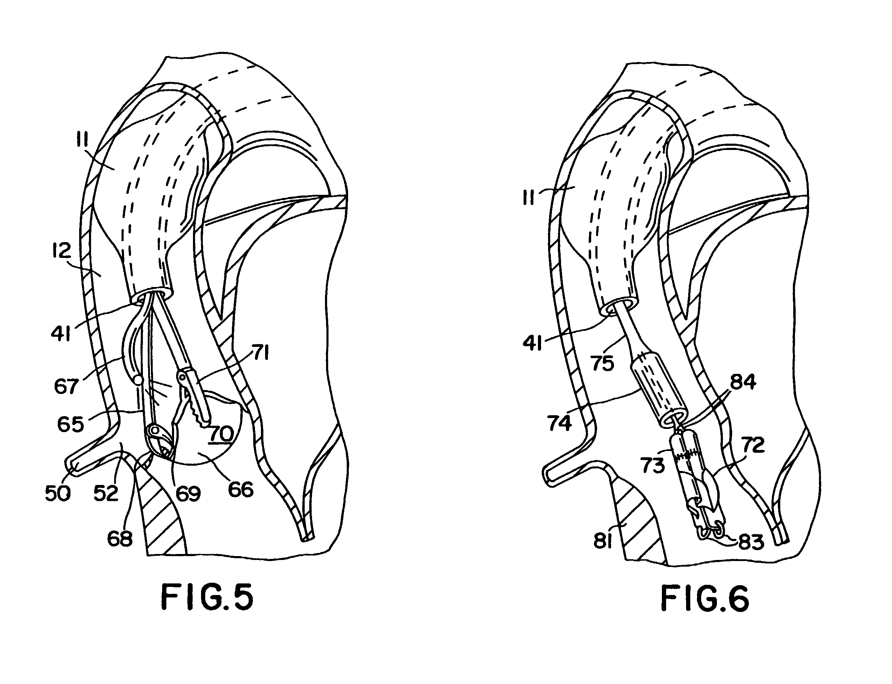

The cardiac accessing system of the invention is particularly useful in the removal of the aortic heart valve and replacement thereof with a prosthetic heart valve which is illustrated in Figs. 5 through 8. As shown in. Fig. 5, a tissue cutter 65 is inserted into the patient through the inner lumen 40 of the occluding catheter 10 and advanced therein to the site of the aortic valve 66 which is to be removed. An angioscope 67 is likewise advanced through the inner lumen 40 until the distal end thereof extends out of the distal end of the occluding catheter 10. At least one of the cutting blades 68 and 69 on the tissue cutter 65 is actuated from the proximal end thereof which extends out of the second arm 30 of the adapter 26 on the proximal end of the catheter 10. The guidance and operation of the cutter 65 is controlled by the physician or other operator while observing the cutter through the angioscope 67. Due to its size and condition, the aortic valve 66 will usually have to be cut into smaller sections, such as section 70 as shown, so that it will fit within the inner lumen 40 of the occluding catheter 10 in order to remove the valve material from the patient.

Forceps 71 or other suitable grasping means are employed to hold onto the aortic valve sections as they are severed by the cutting means 65 to ensure that the valve sections are accurately severed from the site with little or no damage to the underlying tissue of the ascending aorta and removed through the inner lumen 40. The cutting means 65 may have to be withdrawn from the occluding catheter 10 before large severed portions of the aortic valve 66 can be removed by forceps 71. During the procedure a continuous flow of clear liquid, such as the clear cardioplegic fluid exiting the ostia 52 and 53 and/or fluid being infused via the clamp 10 or an angioscope 67, is maintained to facilitate the observation of the region by the operator using the angioscope 67. After the valve 66 has been severed and removed from the region, the instruments used for this particular procedure are withdrawn from the patient through the inner lumen 40 of the occluding catheter 10. Instead of or in addition to mechanical cutting means, laser, electrosurgery, or other cutting methods may be employed in the valve removal procedure.

Direct observation of the placement of the cutting device 65 by suitable imaging means such as an angioscope 67 will ensure accurate positioning of the cutter blades 68 and 69 against the aortic valve to more effectively sever the valve 66 with little or no damage to the supporting aortic tissue. Aortic damage might interfere with the placement of a replacement valve 72 at the site. The precision of the valve removal and replacement is important to the success of endovascular valve replacement. There are several imaging techniques presently available, in addition to the angioscopic technique described, which provide complementary options to assure this precision, namely 1) transesophageal echocardiography; 2) intravascular ultrasound passed through the inner lumen of the delivery catheter 10; 3) intravascular ultrasound or angioscopy passed intravascularly via the venous system through the intra-atrial septum, across the mitral valve, and into the left ventricle; and 4) fluoroscopy. Note that an angioscope within the left ventricle would provide both the added benefit of allowing constant high definition imaging of the entire procedure and high-flow irrigation.

After the heart valve 66 is removed, a replacement valve 72 is then advanced through the inner lumen 40 of the occluding catheter 10 as shown in Fig. 6. The valve 72 is preferably a bioprosthetic valve such as xenograft valve. Porcine glutaraldehyde preserved valves are quite suitable because, as previously mentioned, they are readily accessible, they are storable, and they are available in a variety of sizes. The replacement valve 72, which is shown in Fig. 6 in an inverted and folded condition, has a Dacron skirt 73 secured to the lower rim of the natural porcine valve to facilitate securing the replacement valve to the wall of ascending aorta 12 at or near to the site from which the original aortic valve 66 was removed. The folded and inverted replacement valve 72 is disposed within the expanded end 74 of valve delivery catheter 75 so that the valve 72 can be advanced through the occluding catheter 10. The valve 72 is urged out of the expanded end 74 by the connector cables 84 which are connected to the upper extensions of the valve by releasable means 83. Once outside of the expanded end 74, the valve 72 expands due to the natural resiliency of the valve and the connector cables. The valve delivery catheter 75 is then removed by withdrawing it through the inner lumen 40 of the occluding catheter 10. Alternatively, the valve 72 may be provided with a temporary or permanent expandable support frame. The frame may contain stapling elements to secure the valve to the aortic wall.

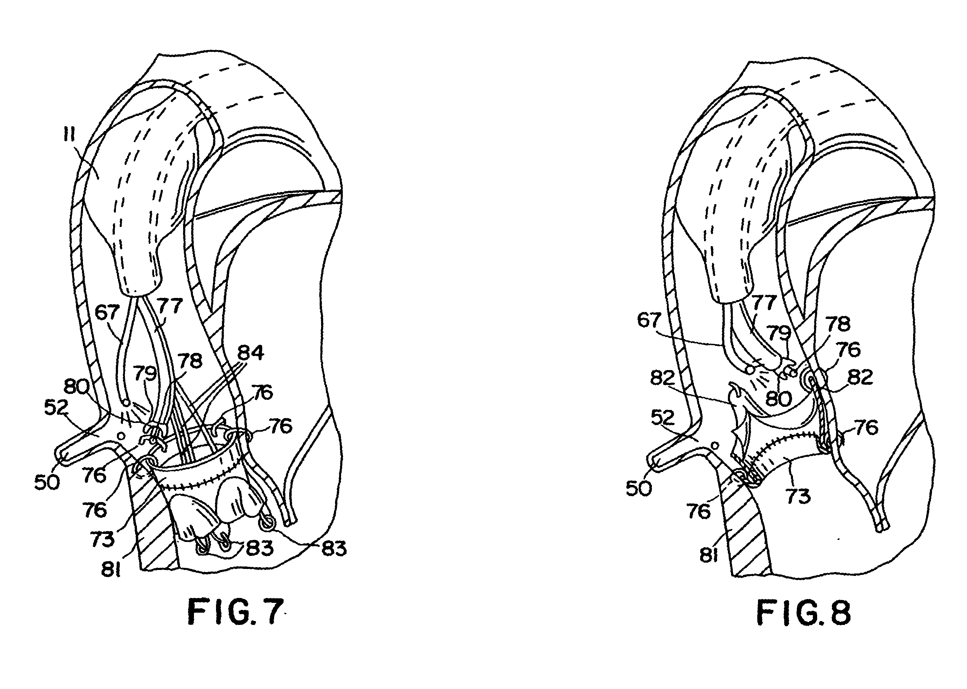

The Dacron skirt 73 is fixed to the aortic root 12 by means of a plurality of staples 76, as shown in Fig. 7, which are secured by the stapling mechanism 77 which is advanced through the inner lumen 40 and out of the distal port 41. The stapling mechanism 77 has an L-shaped holding arm 78 that holds the staple 76 and shaping member 79 having a U-shaped arcuate shaping surface 80 which presses the staple 76 against holding arm 78 deforming the staple as it is pushed through the Dacron skirt 73 and into the aortic wall 81 as shown to force the pointed arms or tines thereof toward each other and fix the staple within the aortic wall. In the alternative the holding arm 78 may be moved toward the shaping member 79 or both may be advanced toward each other. The stapling mechanism 77 is preferably provided with a removable protective sheath (not shown) to facilitate the advancement of the mechanism through the inner lumen 40 without the pointed ends or tines of the staples 76 sticking into the inner wall of the occluding catheter 10 which defines the inner lumen 40. Usually about 10 to about 20 staples will be required to adequately secure the skirt 73 to the aortic wall 81. The angioscope 67 is provided to allow the physician to observe the procedure and guide the stapling mechanism 77 to the desired location and to secure the staple 76 and the skirt 73 at the desired location within the aortic root 12.

Once the Dacron skirt 73 is properly secured, the inverted valve 72 is pulled through the fixed Dacron skirt 73, as shown in Fig. 8, and the upper extensions of the new valve 72 are stapled in essentially the same manner as the Dacron skirt 73. Care must be exercised when placing the Dacron skirt 73 prior to securing it to the aortic wall 81 so that when the inverted portion of the new valve 72 is pulled through the secured Dacron skirt 73, the ostia 52 and 53 of the coronary arteries 50 and 51 are not blocked by the upper extensions 82 of the valve 72. After the upper extensions 82 are secured to the aortic wall 81, the releasable means 83 at the end of the connector cables 84 are released and the cables are withdrawn through the inner lumen 40 of the occluding catheter 10.

Any tissue debris resulting from the aortic valve removal and new valve placement is trapped by the barrier formed by the inflated balloon 11 on the distal end of the occluding catheter 10. However, liquid in the aortic region containing such debris may be removed through an aspiration tube (not shown) disposed within the inner lumen 40 of the occluding catheter 10 or through inner lumen 40 by aspirating the fluid containing the debris. An irrigation catheter may be used to dislodge any debris caught between the inflated balloon 11 and the aortic wall where the two meet.

When the replacement valve 72 is secured in place, the fluid pumped through the retroperfusion catheter 20 is changed to a compatible fluid, *e.g.* saline or blood, containing no cardioplegic agents in order to flush out the cardioplegic materials from the myocardium through the ostia 52 and 53. The pulmonary venting catheter 54 may also be removed at the same time. Shortly thereafter the heart begins to beat on its own or it is externally defibrillated and the blood coming into the right heart is pumped through the pulmonary trunk to the lungs where it is oxygenated in the normal fashion. Oxygenated blood is returned from the lungs into the left atrium and is then pumped from the left ventricle through the new valve into the ascending aorta. Initially, the balloon 11 is maintained in the inflated condition, forcing the blood pumped out of the left ventricle to pass through the region of the ascending aorta 12 into inner lumen 40 of the occluding catheter 10 taking with it debris, emboli and the like. The blood passing through inner lumen 40 is directed through the third arm 32 of adapter 26, through the valve 34 and line 36 leading to blood filter and recovery unit 37 where the blood may be filtered and returned to the patient through the cardiopulmonary by-pass system 18. Alternatively, the position of the valve 34 may be changed by means of arm 85 to discharge blood or other fluid containing tissue, emboli, debris and the like through discharge line 35. After sufficient time has elapsed to ensure that debris and embolus free oxygenated blood is being pumped out of the left ventricle 13 the balloon 11 is deflated to allow natural blood flow through the aorta and the cardiopulmonary by-pass system 18 is shut down.

The occluding catheter shaft 39 may be formed of conventional materials such as polyethylene, polyvinyl chloride and the like. Balloon 11 may be formed of materials such as latex, silicone, C-Flex. or the like. Preferably, the balloon 11 is elastic, so as to expand to and circumferentially occlude the vessel into which it is positioned when fluid pressure is applied to the balloon. Alternatively, the balloon 11 may be formed of polymers such as polyethylene, polyethylene teraphthalate, or a polyolefinic ionomer such as Surlyn®, which is available from E.I. DuPont, DeNemours & Co. Such a balloon would be relatively inelastic when inflated, so as to inflate to a predetermined size and maintain essentially that size even when additional fluid pressure is applied within the interior of the balloon. The balloon 11 will generally have an expanded diameter of about 20 to 40 mm to effectively occlude the patient's ascending aorta and an expanded length of about 2 to about 10 cm so as to be disposed between the coronary ostia and the brachiocephalic artery without blocking these arteries. The overall length of the occluding catheter should be at least 80 cm to facilitate passage through the femoral or brachiocephalic arteries to the ascending aorta.

The retroperfusion catheter 20 may be a commercially available retroperfusion catheter. There are suitable cardiopulmonary by-pass systems available commercially. For a brief discussion of cardiopulmonary by-pass systems reference is made to Weber, John G., Encyclopedia of Medical Devices and Instrumentation, Vol. 3, pp. 1440-1457.

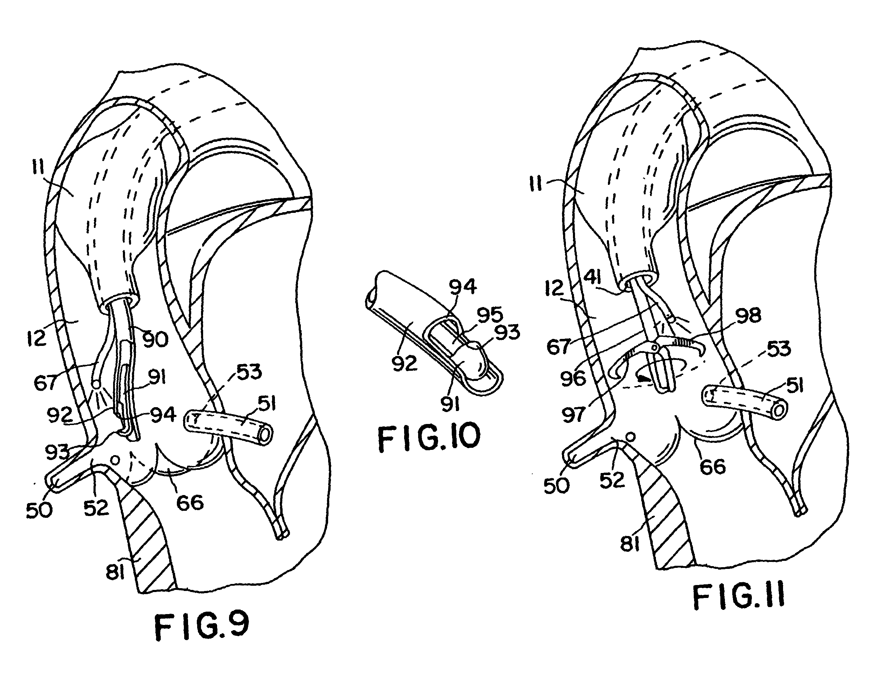

An alternative tissue cutting system is depicted in Figs. 9 and 10. In this embodiment catheter 90 is provided with a cutting head 91 which is slidably disposed within the cutter housing 92. The cutting head 91 is provided with a cutting edge 93 and cutter housing 92 is provided with cutting edge 94. The distal end of the catheter 90 is urged against tissue which is to be removed so that the tissue is pressed into the receiving chamber 95 within the cutting head 91. The cutting head 91 is slidably withdrawn from the cutter housing 92 so that the cutting edge 93 slides by the cutting edge 94 in a cutting relationship so as to sever the tissue within the receiving chamber 95. The severed tissue may be removed by aspiration or the cutting head 91 may be withdrawn from the patient and the severed tissue may be manually or otherwise removed. Preferably, the positioning of the distal end of catheter 90 and the urging of the cutting head against the tissue to be removed is observed by the physician or other operator through angioscope 67 or other suitable imaging system as previously described.

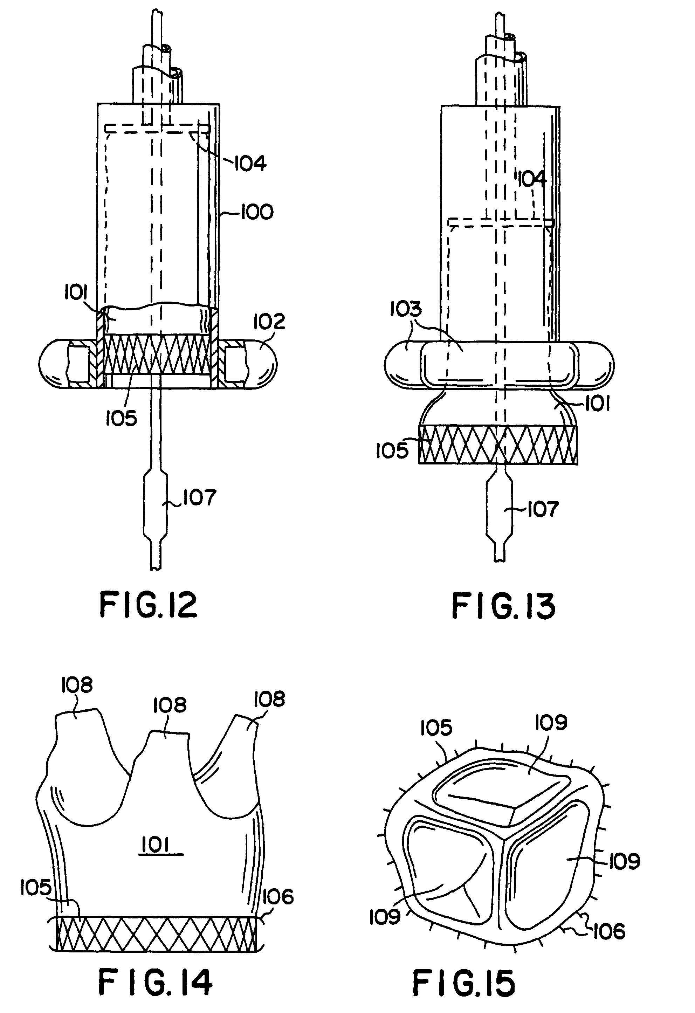

Another cutting system 96, which is shown in Fig. 11, has expandable cutting blades 97 and 98 which are biased or otherwise adapted to expand to a cutting position as shown and rotated at high rotational speeds by a drive shaft and then pressed against the tissue to be severed. The blades 97 and 98 may be biased to expand outwardly by a spring (not shown) or the blades may be forced outwardly by the high speed rotation thereof. This cutting operation is likewise preferably observed by the physician or other operator to ensure proper cutting of the tissue to be removed.

An alternative valve introducer device 100 is shown in Figs. 12-13 which is adapted to contain a prosthetic or replacement valve 101 within expanded distal portion 102. The introducer device 100 is introduced through the inner lumen of the occluding delivery catheter such as previously described until the enlarged distal portion 102 is located at or extends out of the distal end of the delivery catheter. The valve introducer device 100 may be provided with one or more positioning balloons 103 surrounding the expanded distal end 102 thereof which may be inflated in a differential manner, to assure accurate positioning of a prosthetic valve 101 when delivered out of the expanded distal end. A means, such as piston 104 is provided to push the replacement valve 101 out of the expanded distal end 102 when it is in the appropriate position within the patient's ascending aorta. Forceps or other holding means as previously described may be used to position the replacement valve 101 within the location from which the original valve has been removed.

An alternative replacement or prosthetic valve 101 is best shown in the expanded condition in Figs. 14 and 15. As indicated, the valve 101 is provided with a cylindrical base 105 having mounting staples 106 which can be pressed into the wall portion of the ascending aorta at the desired situs by means of an expandable inelastic balloon 107 which is inflated within the valve 101. The upper extensions 108 of the replacement valve 101 from which the leaves or cusps 109 are supported are for the most part self supporting and may not require securing to the wall section of the ascending aorta. The valve introducer device 100 and the inflatable balloon 107 which when inflated presses the mounting staples 106 into the aortic wall may, when deflated, be withdrawn through the inner lumen of a delivery catheter. The aortic region between the site of the replacement valve and the delivery catheter may be well irrigated to remove debris, emboli and the like before regular blood flow through the region is resumed.

The invention provides several benefits, including the ability to endovascularly replace existing cardiac valves or perform other cardiac procedures while avoiding the riskier, more expensive and more traumatic open-heart surgical procedure.

The replacement prosthetic valve device is preferably a bioprosthetic device because these valves do not require the patient to undertake life-long anticoagulant therapy as do mechanical valves. Once inserted, the bioprosthetic valve is capable of operating autonomously. The useful life of a bioprosthetic valve placed via the endovascular procedure may extend to over twenty years, and since most of the valve procedures are performed on the elderly, the bioprosthetic valve will usually function well throughout the remaining life of the patient.

Once the endovascular implantation of the prosthetic valve device is completed in the patient, the function of the prosthetic valve device can be monitored by the same methods as used to monitor valve replacements done by open-heart surgery. Routine physical examination, angiography, or periodic echocardiography can be performed. In contrast to open-heart surgery, however, the patient will recover in a very short period when his or her aortic valve is endovascularly removed and replaced with a prosthetic valve. The replacement valve device can be used in any patient where bioprosthetic valves are indicated, and is particularly suitable for elderly patients and patients unable to tolerate open-heart procedures or life-long anticoagulation.

Unless described otherwise, the various components of the system of the present invention can be formed of conventional materials using conventional manufacturing techniques. The dimensions of the various components are selected so that they perform their intended functions in their intended environment.

While the present invention has been described herein in terms of certain preferred embodiments, it will be apparent to one of ordinary skill in the art that many modifications and improvements can be made to the invention which is defined in the appended claims.

## Claims

1. A system for replacing a heart valve within a patient's aortic region downstream from the patient's left ventricle, comprising:
a) an elongated aortic occlusion catheter (10) adapted to be advanced through a patient's arterial system to a location within the patient's ascending aorta and having proximal (25) and distal ends, expandable means (11) for occluding the ascending aorta on the distal end of the aortic occlusion catheter to position the distal end of the catheter within the patient's aorta, an inner lumen (40) which extends within the catheter to a distal port (41) in the distal end thereof;
b) means for inducing cardioplegia by directing liquid through the patient's coronary blood vessels;
c) means (54) to vent the patient's pulmonary trunk (56); and
d) a cardiopulmonary by-pass system (18) having means to withdraw blood from the patient's venous system, means to remove CO₂ from the withdrawn venous blood, means to oxygenate the withdrawn venous blood and means to return the oxygenated blood to the patient's arterial system,
the system further including a prosthetic valve (72,101), means (75,100) to advance the prosthetic valve, cutting means (65) to sever at least part of the aortic valve from the aortic root and means to advance the cutting means, grasping means (71) for holding the aortic valve as it is being severed by the cutting means, **characterised in that** the valve (72, 101), cutting means and grasping means being advanceable to the distal end of the aortic occlusion catheter (10) through the lumen (40) thereof and out of the distal port (41),

2. The system of claim 1 including a perfusion catheter (20) having proximal and distal ends and an occluding means (47) on the distal end adapted to expand and thereby occlude the patient's coronary sinus and having an inner lumen adapted to deliver in a retrograde manner a fluid containing cardioplegic material through the patient's coronary sinus and coronary veins into the patient's myocardium.

3. The system of claim 1, wherein the vent means (54) is configured to endovascularly pass through the patient's tricuspid (57) and pulmonary (58) valves.

4. The system of claim 3 wherein the means to vent the pulmonary trunk is an elongated catheter (54) which has an inner lumen extending therein between a port in a distal end and a port in a proximal end adapted to extend out of the patient.

5. The system of claim 1 further comprising means for removing all or part of the aortic valve severed from the aortic root through the inner lumen (40) of the aortic catheter.

6. The system of claim 1 or claim 5 including scope means (67) to image the site of the aortic heart valve to allow an operator to visualize the site and accurately urge the valve removal means into engagement with the patient's aortic valve.

7. The system of any one of the preceding claims wherein the prosthetic valve (72,101) has a cylindrical base (73,105) and a plurality of upper extensions (82,108) extending away from the base, and is advanced through the lumen (40) with the upper extensions thereof extending distally.

8. The system of claim 7 wherein the means to advance the prosthetic valve (72,101) through the inner lumen includes high strength elongated members (84) which are releasably secured to the upper extensions (82,108) of the prosthetic valve and which are adapted to be withdrawn to pull the upper extensions of the prosthetic valve through the cylindrical base to revert the valve to its proper position within the aortic region.

9. The system of claim 8 wherein the means to secure the prosthetic valve in the aortic region downstream from the patient's left ventricle includes means (76) to secure the cylindrical base of the prosthetic valve to a wall portion (81) of the aortic region or aortic root.

10. The system of claim 9 wherein the means to secure the cylindrical base of the prosthetic valve is a plurality of staples (76).

11. The system of claim 10 wherein the staples are generally U-shaped and have a pair of pointed tines adapted to be advanced through the cylindrical base of the prosthetic valve and into the wall section of the ascending aorta and to be deformed so as to be secured with the wall section (81) of the ascending aorta.

12. The system of claim 10 including means to secure the upper extensions of the prosthetic valve to a wall portion of the aortic region.

13. The system of claim 11 wherein the means to secure the upper extensions are staples (76).

14. The system of claim 13 including stapling means (77) having an L-shaped staple holder (78) and a U-shaped staple former (80) one or both of which are adapted to be urged toward the other to plasticly deform the staple so that the tines are pressed toward one another and secured within the wall section (81) of the aortic region.

15. The system of claim 1 wherein the prosthetic valve (101) has a supporting wire frame (105).

## Patentansprüche

1. System, um eine Herzklappe innerhalb einer Aortengegend eines Patienten stromab der linken Herzkammer des Patienten zu ersetzen, umfassend:
a) einen länglichen Aorten-Verschlusskatheter (10), der zum Vorwärtsbewegen durch das Arteriensystem eines Patienten zu einer Position innerhalb der aufsteigenden Aorta des Patienten ausgebildet ist und ein proximales (25) und ein distales Ende, ein expandierbares Mittel (11) zum Verschließen der aufsteigenden Aorta am distalen Ende des Aorten-Verschlusskatheters, um das distale Ende des Katheters innerhalb der Aorta des Patienten zu positionieren, sowie ein Innenlumen (40) aufweist, das sich innerhalb des Katheters zu einer distalen Öffnung (41) in dessen distalem Ende erstreckt;
b) Mittel zum Herbeiführen von Kardioplegie durch das Leiten von Flüssigkeit durch die koronaren Blutgefäße des Patienten;
c) Mittel (54) zur Ventilation des Pulmonalstamms (56) des Patienten; und
d) ein Herz-Lungen-Bypass-System (18), das Mittel zum Entnehmen von Blut aus dem Venensystem des Patienten, Mittel zum Entfernen von CO₂ aus dem entnommenen venösen Blut, Mittel zur Anreicherung des entnommenen venösen Bluts mit Sauerstoff und Mittel zum Zurückführen des mit Sauerstoff angereicherten Bluts zum Arteriensystem des Patienten umfasst,
wobei das System weiters eine Klappenprothese (72, 101), Mittel (75, 100) zum Vorwärtsbewegen der Klappenprothese, Schneidmittel (65) zum Abtrennen zumindest eines Teils der Aortenklappe vom Aortenstamm und Mittel zum Vorwärtsbewegen des Schneidmittels sowie Greifmittel (71) umfasst, um die Aortenklappe zu halten, während sie vom Schneidmittel abgetrennt wird, **dadurch gekennzeichnet, dass** die Klappe (72, 101), das Schneidmittel und das Greifmittel zum distalen Ende des Aorten-Verschlusskatheters (10) durch dessen Lumen (40) und aus der distalen Öffnung (41) hinaus vorwärts bewegt werden können.

2. System nach Anspruch 1, das einen Perfusionskatheter (20) umfasst, der ein proximales und ein distales Ende sowie ein Verschlussmittel (47) am distalen Ende umfasst, das dazu ausgebildet ist, sich auszuweiten, wodurch der Koranarsinus des Patienten verschlossen wird, und der ein Innenlumen aufweist, das zur retrograden Abgabe eines Fluids, das Kardioplegiematerial enthält, durch den Koronarsinus und die Koronarvenen des Patienten in den Herzmuskel des Patienten ausgebildet ist.

3. System nach Anspruch 1, worin das Ventilationsmittel (54) so konfiguriert ist, dass es endovaskulär durch die Trikuspidalklappe (57) und die Pulmonalklappe (58) des Patienten hindurchgeht.

4. System nach Anspruch 3, worin das Mittel zur Ventilation des Pulmonalstamms ein länglicher Katheter (54) ist, der ein Innenlumen aufweist, das sich in diesem zwischen einer Öffnung im distalen Ende und einer Öffnung im proximalen Ende erstreckt, der dazu ausgebildet ist, sich aus dem Patienten heraus zu erstrecken.

5. System nach Anspruch 1, das weiters Mittel zum Entfernen der gesamten oder eines Teils der vom Aortenstamm abgetrennten Aortenklappe durch das Innenlumen (40) des Aortenkatheters aufweist.

6. System nach Anspruch 1 oder 5, das Betrachtungsmittel (67) zum Abbilden der Lage der Aortenherzklappe umfasst, um es einer Bedienperson zu ermöglichen, die Lage zu betrachten und das Klappen-Entfemungsmittel präzise in Eingriff mit der Aortenklappe des Patienten zu bringen.

7. System nach einem der vorangegangenen Ansprüche, worin die Klappen-Prothese (72, 101) eine zylindrische Basis (73, 105) und eine Vielzahl oberer Fortsätze (82, 108) aufweist, die sich von der Basis weg erstrecken, und durch das Lumen (40) vorwärts bewegt wird, wobei sich deren obere Fortsätze distal erstrecken.

8. System nach Anspruch 7, worin das Mittel zum Vorwärtsbewegen der Klappen-Prothese (72, 101) durch das Innenlumen längliche Elemente (84) mit hoher Festigkeit umfasst, die abnehmbar an den oberen Fortsätzen (82, 108) der Klappen-Prothese befestigt sind und dazu ausgebildet sind, zurückgezogen zu werden, um die oberen Fortsätze der Klappen-Prothese durch die zylindrische Basis zu ziehen, um die Klappe wieder in ihre richtige Position innerhalb der Aortengegend zu bringen.

9. System nach Anspruch 8, worin das Mittel zum Befestigen der Klappen-Prothese in der Aortengegend stromab der linken Herzkammer des Patienten Mittel (76) zum Befestigen der zylindrischen Basis der Klappen-Prothese an einem Wandabschnitt (81) der Aortengegend oder des Aortenstamms umfasst.

10. System nach Anspruch 9, worin das Mittel zum Befestigen der zylindrischen Basis der Klappen-Prothese aus einer Vielzahl von Klammern (76) gebildet ist.

11. System nach Anspruch 10, worin die Klammern im Allgemeinen U-förmig sind und ein Paar spitzer Zinken aufweisen, die dazu ausgebildet sind, durch die zylindrische Basis der Klappen-Prothese und in den Wandabschnitt der aufsteigenden Aorta vorwärts bewegt zu werden und so verformt zu werden, dass sie am Wandabschnitt (81) der aufsteigenden Aorta befestigt werden.

12. System nach Anspruch 10, das Mittel zum Befestigen der oberen Fortsätze der Klappen-Prothese an einem Wandabschnitt der Aortengegend umfasst.

13. System nach Anspruch 11, worin die Mittel zum Befestigen der oberen Fortsätze Klammern (76) sind.

14. System nach Anspruch 13, das Heftmittel (77) mit einer L-förmigen Klammer-Halterung (78) und einem U-förmigen Klammer-Former (80) umfasst, wobei eines davon oder beide dazu ausgebildet sind, auf einander zu bewegt zu werden, um die Klammer plastisch zu verformen, so dass die Zinken auf einander zu gedrückt und innerhalb des Wandabschnitts (81) der Aortengegend befestigt werden.

15. System nach Anspruch 1, worin die Klappen-Prothese (101) einen stützenden Drahtrahmen (105) aufweist.

## Revendications

1. Un système pour remplacer une valve cardiaque dans la région aortique d'un patient, en aval du ventricule gauche du patient, comprenant :
a) un cathéter d'occlusion aortique (10) allongé, adapté pour être avancé dans un système artériel d'un patient jusqu'à un emplacement à l'intérieur de l'aorte montante du patient et ayant des extrémités proximales (25) et distales, des moyens expansibles (11) pour obturer l'aorte montante sur l'extrémité distale du cathéter d'occlusion aortique, afin de positionner l'extrémité distale du cathéter à l'intérieur de l'aorte du patient, un lumen intérieur (40) s'étendant à l'intérieur du cathéter, jusqu'à un orifice distale (41) ménagé dans son extrémité distale ;
b) des moyens pour induire une cardioplégie en dirigeant du liquide dans les vaisseaux sanguins coronaires du patient ;
c) des moyens (54) pour aérer l'artère pulmonaire (56) du patient ; et
d) un système de dérivation cardiopulmonaire (18), comportant des moyens pour extraire du sang du système veineux du patient, des moyens pour extraire le CO₂ du sang veineux ayant été extrait, des moyens pour oxygéner le sang veineux ayant été extrait, et des moyens pour retourner le sang oxygéné au système artériel du patient,
le système comprenant en outre une valve prothétique (72,101), des moyens (75,100) pour faire progresser la valve prothétique, des moyens de découpage (65) pour séparer au moins une partie de la valve aortique vis-à-vis de l'extrémité inférieure de la crosse de l'aorte et des moyens pour faire avancer les moyens de découpage, des moyens de saisie (71) pour tenir la valve aortique au moment où elle est séparée par les moyens de découpage, **caractérisé en ce que** la valve (72,101), les moyens de découpage et les moyens de saisie sont susceptibles d'être avancés jusqu'à l'extrémité distale du cathéter d'occlusion aortique (10) en passant par son lumen (40) et en sortant de l'orifice distal (41).

2. Le système selon la revendication 1, comprenant un cathéter de perfusion (20) comportant des extrémités proximales et distales et des moyens d'occlusion (47) sur l'extrémité distale, adaptés pour expanser et, de cette manière, occlure le sinus coronaire du patient et comportant un lumen intérieur adapté pour délivrer de manière rétrograde un fluide contenant du matériau cardioplégique par le sinus coronaire du patient et les veines coronaires dans le myocarde du patient.

3. Le système selon la revendication 1, dans lequel les moyens d'aération (54) sont configurés pour un passage endovasculaire à travers les valves tricuspides (57) et pulmonaires (58).

4. Le système selon la revendication 3, dans lequel les moyens pour aérer l'aorte pulmonaire sont formés d'un cathéter (54) allongé ayant un lumen intérieur s'y étendant entre un orifice placé dans une extrémité distale et un orifice placé dans une extrémité proximale adaptée pour s'étendre hors du patient.

5. Le système selon la revendication 1, comprenant en outre des moyens pour supprimer la totalité ou une partie de la valve aortique ayant été séparée de l'extrémité inférieure de la crosse de l'aorte, par le lumen intérieur (40) du cathéter aortique.

6. Le système selon la revendication 1 ou la revendication 5, comprenant des moyens d'imagerie (67) pour imager le site de la valeur cardiaque aortique, pour permettre à un opérateur de visualiser le site et de déplacer de façon précise les moyens d'enlèvement de valve, en prise avec la valve aortique du patient.

7. Le système selon l'une quelconque des revendications précédentes, dans lequel la valve prothétique (72,101) comporte une base (73,105) cylindrique et une pluralité d'extensions supérieures (82,108) s'écartant de la base et est avancée à travers le lumen (40), ses extensions supérieures s'étendant de façon distale.

8. Le système selon la revendication 7, dans lequel les moyens permettant de faire avancer la valve prothétique (72,101) à travers le lumen intérieur comprennent des organes (84) allongés, de haute résistance, fixés de façon désolidarisable aux extensions supérieures (82,108) de la valve prothétique et adaptés pour être extraits pour tirer des extensions supérieures de la valve prothétique à travers la base cylindrique, pour ramener la valve à sa position correcte à l'intérieur de la région aortique.

9. Le système selon la revendication 8, dans lequel les moyens permettant de fixer la valve prothétique dans la région aortique, en aval du ventricule gauche du patient, comprennent les moyens (76), permettant de fixer la base cylindrique de la valve prothétique à une partie de paroi (81) de la région aortique ou de l'extrémité inférieure de la crosse de l'aorte.

10. Le système selon la revendication 9, dans lequel les moyens permettant de fixer la base cylindrique de la valve prothétique sont formés par une pluralité d'agrafes (76).

11. Le système selon la revendication 10, dans lequel les d'agrafes sont globalement en forme de U et comportent une paire de dents pointues, adaptées pour être avancées à travers la base cylindrique de la valve prothétique et dans la section de paroi de l'aorte montante et pour être déformées pour être fixées à la section de paroi (81) de l'aorte montante.

12. Le système selon la revendication 10, comprenant des moyens pour fixer les extensions supérieures de la valve prothétique à une partie de paroi de la région aortique.

13. Le système selon la revendication 11, dans lequel les moyens de fixation des extensions supérieures sont des agrafes (76).

14. Le système selon la revendication 13, comprenant des moyens d'agrafage (77) ayant un support d'agrafe (78) en forme de L et un formeur d'agrafe (80) en forme de U, dont l'un ou les deux sont adaptés pour être déplacés vers l'autre pour déformer plastiquement l'agrafe, de manière que les dents soient pressées les unes vers les autres et fixées à l'intérieur de la section de paroi (81) de la région aortique.

15. Le système selon la revendication 1, dans lequel la valve prothétique (101) comporte un cadre de support (105) en fil.
